# EUROPÄISCHE PATENTSCHRIFT

(11) **EP 4 048 328 B1**
(45) Veröffentlichungstag und Bekanntmachung des Hinweises auf die Patenterteilung: **08.10.2025**
(21) Anmeldenummer: 20796788.6
(22) Anmeldetag: 22.10.2020
(51) Int. Cl.: A61L 15/42, A61L 15/44, B41J 2/01

(54) **WUNDAUFLAGE UMFASSEND EINE WUNDKONTAKTSCHICHT MIT EINER BAHNFÖRMIGEN BESCHICHTUNG FÜR EINE GERICHTETE ZELLMIGRATION**
WOUND DRESSING COMPRISING A WOUND CONTACT LAYER HAVING A WEB-LIKE COATING FOR DIRECTIONAL CELL MIGRATION
PANSEMENT COMPRENANT UNE COUCHE DE CONTACT AVEC LA PLAIE PRÉSENTANT UN REVÊTEMENT DE TYPE BANDE POUR UNE MIGRATION CELLULAIRE DIRECTIONNELLE

(30) Priorität: 23.10.2019 DE 102019128569
(43) Veröffentlichungstag der Anmeldung: 31.08.2022
(73) Patentinhaber: Paul Hartmann AG, 89522 Heidenheim (DE)
(72) Erfinder: KETTEL, Markus, 89520 Heidenheim (DE); JÜNGST, Tomasz, 97072 Würzburg (DE); GROLL, Jürgen, 97246 Eibelstadt (DE)
(74) Vertreter: Paul Hartmann AG Patents & Licensing
(86) Internationale Anmeldenummer: PCT/EP2020/079774
(87) Internationale Veröffentlichungsnummer: WO 2021/078871

(56) Entgegenhaltungen:
- US-A1- 2011 189 287
- SCOUTARIS NICOLAOS ET AL: "Current Trends on Medical and Pharmaceutical Applications of Inkjet Printing Technology", PHARMACEUTICAL RESEARCH, SPRINGER NEW YORK LLC, US, vol. 33, no. 8, 12 May 2016 (2016-05-12), pages 1799 - 1816, XP036002063, ISSN: 0724-8741, [retrieved on 20160512], DOI: 10.1007/S11095-016-1931-3

## Beschreibung

Die vorliegende Erfindung betrifft eine Wundauflage umfassend eine Wundkontaktschicht, wobei eine wundzugewandte Seite der Wundkontaktschicht eine Beschichtung aufweist. Die Erfindung betrifft auch ein Verfahren zur Herstellung der zuvor genannten Wundauflage.

Wundauflagen mit einer eine Beschichtung aufweisenden Wundkontaktschicht sind im Stand der Technik bekannt. Die Beschichtung kann der Wundkontaktschicht eine bestimmte Eigenschaft, zum Beispiel eine antibakterielle Wirksamkeit im Falle einer Silberbeschichtung oder eine nicht mit der Wunde verklebende Oberfläche im Falle einer Salbenimprägnierung oder einer Silikonbeschichtung, verleihen. Bei den zuvor genannten Beispielen wird die Beschichtung oftmals vollflächig, flächig mit Öffnungen oder punktförmig auf der Wundkontaktschicht aufgetragen.

Wundauflagen mit einer Wundkontaktschicht aus einem Hydrogel sind ebenfalls im Stand der Technik bekannt. Derartige Wundauflagen können sowohl Wundexsudat absorbieren als auch die Wunde befeuchten. Wundauflagen mit einer Hydrogel-Wundkontaktschicht haben in der modernen Wundbehandlung einen hohen Stellenwert erlangt. Die Weiterentwicklung dieser bewährten Technologie ist ein Anliegen von sowohl den Anwendern als auch der Industrie.

Die US 2011/0189287 A1 offenbart einen Wundverband, welcher ein Trägermaterial mit einer zu einer Wunde ausgerichteten Oberfläche umfasst, wobei die zur Wunde ausgerichtete Oberfläche mit einem speziellen Matrixmaterial modifiziert ist. Beim Aufbringen auf eine Wunde wird die Oberfläche des mit dem Matrixmaterial modifizierten Trägermaterials in Kontakt mit dem Wundbett gebracht. Das Matrixmaterial kann einen Wirkstoff enthalten. Es soll das Wundbett verändern und damit eine homogene Umgebung schaffen, die eine gleichmäßige und vorhersehbare Abgabe oder gleichmäßige Aufnahme von Wirkstoffen in das Wundbett ermöglicht.

Aufgabe der vorliegenden Erfindung war es, eine Wundauflage zur Verfügung zu stellen, welche gezielt Einfluss auf die Zellen in einer Wunde nehmen kann.

Insbesondere lag der vorliegenden Erfindung die Aufgabe zugrunde, eine Wundauflage bereitzustellen, mit der gezielt Einfluss auf die Zellen in einer Wunde genommen werden kann, um den Wundheilungsprozess positiv beeinflussen zu können, beispielsweise indem der Wundverschluss beschleunigt werden kann.

Eine weitere Aufgabe der vorliegenden Erfindung bestand darin, ein möglichst einfaches und ökonomisches Herstellungsverfahren für eine solche Wundauflage anzugeben.

Zur Lösung dieser Aufgaben wird eine Wundauflage nach Anspruch 1 und ein Verfahren nach Anspruch 2 vorgeschlagen.

Die erfindungsgemäße Wundauflage umfasst eine Wundkontaktschicht, wobei die Wundkontaktschicht ein Hydrogel umfasst oder aus einem Hydrogel besteht und eine wundzugewandte Seite der Wundkontaktschicht eine Beschichtung aufweist. Die Beschichtung kann eine Adhäsion von Zellen (Zelladhäsion) eines Patienten vermitteln. Das heißt, Zellen des Patienten können an der Beschichtung adhärieren beziehungsweise anhaften. Zudem bildet die Beschichtung eine oder mehrere Bahnen auf der wundzugewandten Seite der Wundkontaktschicht aus. Indem die Beschichtung die Zelladhäsion vermitteln kann und bahnförmig ausgestaltet ist, kann die Wundauflage gezielt Einfluss auf die Zellen in einer Wunde nehmen.

Ob ein Material eine Zelladhäsion im Sinne der vorliegenden Erfindung vermitteln kann, lässt sich zum Beispiel mit Hilfe von einem Zellkulturversuch bestimmen. Wenn adhärent wachsende Zellen wie zum Beispiel L929 Fibroblasten in der Zellkultur an dem Material haften bleiben und davon nicht abgewaschen werden können (es folglich als Substrat für die Zellen dienen kann), kann das Material eine Zelladhäsion im Sinne der vorliegenden Erfindung vermitteln.

Das erfindungsgemäße Verfahren zur Herstellung einer Wundauflage umfasst die folgenden Schritte:
i. Bereitstellen einer Wundkontaktschicht, wobei die Wundkontaktschicht ein Hydrogel umfasst oder aus einem Hydrogel besteht.
ii. Beschichten einer wundzugewandten Seite der Wundkontaktschicht, so dass die wundzugewandte Seite der Wundkontaktschicht eine Beschichtung aufweist, wobei die Beschichtung eine Adhäsion von Zellen eines Patienten vermitteln kann und die Beschichtung eine oder mehrere Bahnen auf der wundzugewandten Seite der Wundkontaktschicht ausbildet.

Mit dem Begriff "Zellen" sind vorliegend menschliche oder tierische Zellen gemeint. Mikroorganismen wie insbesondere Bakterien sind mit dem Begriff "Zellen" nicht gemeint. Die zur Adhäsion an die Beschichtung vorgesehenen Zellen stammen bei einer Behandlung einer Wunde eines Menschen oder Tieres mit der erfindungsgemäßen Wundauflage typischerweise von dem behandelten Mensch oder Tier (dem "Patient") selbst.

Die erfindungsgemäße Wundauflage ist zur Behandlung von äußeren beziehungsweise offenen Wunden der Haut vorgesehen. Die Wunde wird mit der Wundauflage abgedeckt. Dabei kommt die wundzugewandte Seite der Wundkontaktschicht mit der Oberfläche der Wunde in Berührung. An der Wundheilung beteiligte Zellen wie beispielsweise Fibroblasten oder Keratinozyten können dann an der Beschichtung haften bleiben und sich entlang der Bahnen der Beschichtung bewegen. Auf diese Art und Weise können die Zellen zum Beispiel von einem Rand der Wunde in das Innere der Wunde geleitet und der Wundverschluss beschleunigt werden. Vereinfacht ausgedrückt kann die bahnförmige Beschichtung der Wundkontaktschicht also als eine Art Straßensystem verstanden werden, auf dem sich an der Wundheilung beteiligte Zellen bewegen sollen und mit dem sie beispielsweise von einem Rand der Wunde in das Innere der Wunde geleitet werden sollen, wodurch der Wundverschluss beschleunigt werden soll. Mit der erfindungsgemäßen Wundauflage wird also versucht, die Bewegung der Zellen, auch Migration genannt, in eine für den Wundheilungsprozess vorteilhafte Richtung zu leiten. Dies erklärt vereinfacht ausgedrückt, wie die Wundauflage gezielt Einfluss auf die Zellen in einer Wunde nehmen kann, um den Wundheilungsprozess positiv zu beeinflussen. Weitere Details der vorliegenden Erfindung können den nachfolgenden bevorzugten Ausführungsformen entnommen werden.

Vorzugsweise kann die unbeschichtete Wundkontaktschicht im Wesentlichen keine Zelladhäsion vermitteln. Dadurch kann der gewünschte Effekt der Beschichtung, nämlich die gerichtete Zellmigration, besser zum Tragen kommen. Zudem kann die Wundkontaktschicht dann weniger stark an der Wundoberfläche anhaften, was einen schonenderen Wechsel der Wundauflage ermöglichen kann.

Erfindungsgemäß umfasst die Wundkontaktschicht ein Hydrogel oder die Wundkontaktschicht besteht aus einem Hydrogel. Eine Hydrogel-Wundkontaktschicht kann sowohl Wundexsudat absorbieren als auch die Wunde befeuchten. Eine Hydrogel-Wundkontaktschicht eignet sich für die schonende Behandlung von vielen unterschiedlichen Wundtypen. Zudem kann eine Hydrogel-Wundkontaktschicht die Eigenschaft im Wesentlichen keine Zelladhäsion zu vermitteln aufweisen.

Ein für die vorliegende Erfindung besonders geeignetes Hydrogel ist in der deutschen Patentanmeldung DE 10 2016 125 534 A1 offenbart. Dieses Hydrogel ist erhältlich durch Umsetzung eines Amin-terminierten Präpolymers enthaltend Polyalkylenoxideinheiten mit einem Isocyanat-terminierten Präpolymer enthaltend Polyalkylenoxideinheiten. Die Umsetzung erfolgt vorzugsweise in Gegenwart eines mehrwertigen Alkohols wie zum Beispiel Ethylenglykol, Glycerol, Sorbitol, PEG300, PEG2000 oder Saccharose. Zudem erfolgt die Umsetzung typischerweise in Gegenwart von Wasser. Weiterhin kann das Isocyanat-terminierte Präpolymer mindestens dreiarmig verzweigt sein und die Polyalkylenoxideinheiten der beiden Präpolymere können durch Polyethylenoxid- und/oder Polypropylenoxideinheiten gebildet werden, wobei das Gewichtsverhältnis von Ethylenoxidzu Propylenoxideinheiten vorzugsweise 3 : 1 bis 7 : 1 beträgt. Ein geeignetes Aminterminiertes Präpolymer ist im Handel unter der Bezeichnung Jeffamin^{®} ED-2003 (Huntsman; Everberg, Belgien) erhältlich. Ein geeignetes Isocyanat-terminiertes Präpolymer ist im Handel unter der Bezeichnung Aquapol^{®} PI-13000-31 (Carpenter; Richmond, USA) erhältlich. Zusätzliche Informationen zur chemischen Struktur von Aquapol^{®} sind in der europäischen Patentanmeldung mit der Anmeldenummer EP 18179037.9 enthalten.

Vorteilhaft im Zusammenhang mit der vorliegenden Erfindung ist es auch, wenn die Wundkontaktschicht durchgängig und/oder im Wesentlichen eben ausgebildet ist. Eine durchgängige Wundkontaktschicht hat eine kontinuierliche Oberfläche ohne Löcher, Schlitze oder ähnliche Öffnungen. Eine derartige Wundkontaktschicht kann leicht und in vielfältiger Weise mit einer bahnförmigen Beschichtung strukturiert werden. Eine Hydrogel-Wundkontaktschicht kann leicht so hergestellt werden, dass sie durchgängig und eben ausgebildet ist.

Erfindungsgemäß ist es vorgesehen, dass die Beschichtung eine Adhäsion von Zellen vermitteln kann und die Beschichtung eine oder mehrere Bahnen auf der wundzugewandten Seite der Wundkontaktschicht ausbildet. Typischerweise führt dies wie bereits erwähnt dazu, dass die Beschichtung eine Migration und/oder Proliferation von Zellen leiten und/oder fördern kann. Bei den zur Adhäsion an die Beschichtung vorgesehenen Zellen kann es sich vorteilhafterweise um an der Wundheilung beteiligte menschliche oder tierische Zellen, insbesondere um Fibroblasten oder Keratinozyten, handeln.

Die Adhäsion von Zellen an die Beschichtung kann dadurch vermittelt werden, indem die Beschichtung einen Alkylrest, einen Alkohol, ein Amin, eine Fettsäure, ein Bestandteil einer extrazellulären Matrix (EZM-Bestandteil), ein denaturierten Bestandteil einer extrazellulären Matrix, ein Gerinnungsfaktor, ein Zelladhäsionsmolekül, ein synthetisches Polymer und/oder einen Zucker umfasst. Aus dieser Gruppe werden der EZM-Bestandteil (nicht denaturiert), der Gerinnungsfaktor und das Zelladhäsionsmolekül bevorzugt, da mit diesen eine besonders spezifische Zelladhäsion vermittelt werden kann.

Der Alkylrest weist vorzugsweise mindestens 8 Kohlenstoffatome, insbesondere mehr als 12 Kohlenstoffatome, auf und ist zudem vorteilhafterweise unverzweigt aufgebaut.

Der Alkohol und das Amin weisen gleichfalls vorzugsweise mindestens 8 Kohlenstoffatome, insbesondere mehr als 12 Kohlenstoffatome, auf. Zudem sind der Alkohol und das Amin bevorzugt aliphatisch und/oder unverzweigt aufgebaut. Zum Beispiel kann der Alkohol Tridecan-1-ol, Tetradecan-1-ol, Pentadecan-1-ol, Hexadecan-1-ol oder Octadecan-1-ol sein. Der Alkohol kann jedoch auch ein Monoglycerid oder ein Diglycerid sein, insbesondere als Ester des Glycerols mit einer oder zwei Fettsäuren gemäß der nachfolgenden bevorzugten Ausführungsformen.

Die Fettsäure ist bevorzugt mittelkettig oder langkettig. Eine mittelkettige Fettsäure weist 8 bis 12 Kohlenstoffatome auf. Eine langkettige beziehungsweise höhere Fettsäure weist mehr als 12 Kohlenstoffatome auf. Vorgesehen sind sowohl gesättigte als auch ungesättigte Fettsäuren, welche bevorzugt unverzweigt aufgebaut sind. Entsprechend kann es sich bei der Fettsäure zum Beispiel um Tridecansäure, Tetradecansäure, Pentadecansäure, Hexadecansäure, Heptadecansäure, Octadecansäure, Ölsäure, Linolsäure oder Linolensäure handeln.

Bei dem Bestandteil einer extrazellulären Matrix kann es sich zum Beispiel um Elastin, Entactin, Fibronectin, Hyaluronsäure, Kollagen, Laminin oder Vitronectin handeln. Besonders bevorzugt werden dabei die beiden Adhäsionsproteine Fibronectin und Vitronectin verwendet. Mit dem Begriff Kollagen ist vorliegend nicht nur Kollagen Typ I, sondern auch jeder andere Kollagentyp gemeint. Ebenso ist mit dem Begriff Laminin nicht nur Laminin-1, sondern auch jede andere Lamininform gemeint. Bei dem denaturierten Bestandteil einer extrazellulären Matrix kann es sich beispielsweise um Gelatine handeln.

Der Gerinnungsfaktor ist vorzugsweise Fibrinogen. Es hat sich gezeigt, dass auch Fibrinogen in vorteilhafter Weise die Adhäsion von Zellen an die Beschichtung vermitteln kann, wenn dieser Gerinnungsfaktor in der Beschichtung enthalten ist.

Zelladhäsionsmoleküle sind Membranproteine, welche mit benachbarten Zellen oder der extrazellulären Matrix in Kontakt treten können. Das Zelladhäsionsmolekül kann ein Cadherin, ein Integrin oder ein Selektin sein. Cadherine sowie Selektine können Zellen miteinander in Kontakt bringen, wohingegen Integrine sowie auch Selektine Zell-Matrix-Kontakte hervorrufen können. Im Zusammenhang mit der vorliegenden Erfindung sind insbesondere diejenigen Zelladhäsionsmoleküle von Vorteil, welche dafür vorgesehen sind mit benachbarten Zellen in Kontakt zu treten. Entsprechend stellen Cadherine und Selektine bevorzugte Zelladhäsionsmoleküle für die vorliegende Erfindung dar.

Als synthetisches Polymer kann insbesondere Polylysin in der Beschichtung enthalten sein. Der Zucker schließlich ist bevorzugt oligomer (Oligosaccharid) oder polymer (Polysaccharid) aufgebaut. Insbesondere handelt es sich bei dem Zucker um ein Polysaccharid.

Die Beschichtung kann auch Derivate oder nur Fragmente, insbesondere die jeweiligen Zellbindungsdomänen, der zuvor genannten Stoffe zur Vermittlung der Zelladhäsion enthalten. Ebenso ist es möglich, dass die Beschichtung mehrere der zuvor genannten Stoffe zur Vermittlung der Zelladhäsion enthält. Zum Beispiel könnte die Beschichtung sowohl Fibronectin als auch Fibrinogen enthalten. Die meisten der zuvor genannten Stoffe zur Vermittlung der Zelladhäsion können ferner natürlichen Ursprungs oder künstlich hergestellt worden sein, zum Beispiel durch einen biotechnologischen Herstellungsprozess.

Wie bereits beschrieben bildet die Beschichtung erfindungsgemäß eine oder mehrere Bahnen auf der wundzugewandten Seite der Wundkontaktschicht aus, um die Zellmigration in eine für die Wundheilung vorteilhafte Richtung leiten zu können. Vorzugsweise verlaufen die eine oder mehreren Bahnen von einem Randbereich der wundzugewandten Seite der Wundkontaktschicht mindestens zu einem mittigen oder zentralen Bereich der wundzugewandten Seite der Wundkontaktschicht. Diese Ausgestaltung wird für den Wundheilungsprozess als vorteilhaft angesehen, weil Zellen dann vom Wundrand in die Wunde geleitet werden können.

Insbesondere können die eine oder mehreren Bahnen ein gitterförmiges, sternförmiges oder strahlenförmiges Muster auf der wundzugewandten Seite der Wundkontaktschicht ausbilden.

Dabei kann das gitterförmige Muster im Wesentlichen parallel zueinander angeordnete und im Wesentlichen geradlinig verlaufende Bahnen umfassen. Das strahlenförmige Muster dagegen kann im Wesentlichen geradlinig verlaufende Bahnen umfassen, wobei die Bahnen um einen im Wesentlichen zentralen Bereich der wundzugewandten Seite der Wundkontaktschicht in unterschiedliche Richtungen weisend angeordnet sind. Vorzugsweise erstreckt sich das Muster über im Wesentlichen die gesamte Fläche der wundzugewandten Seite der Wundkontaktschicht. Das heißt, überwiegend die gesamte Fläche der wundzugewandten Seite der Wundkontaktschicht und nicht etwa nur ein Teilbereich von dieser ist gemustert. Eine Beschichtung mit einem derartigen Muster kann gut für eine gerichtete Zellmigration in die Wunde hinein geeignet sein.

Wie bei der Form beziehungsweise dem Verlauf der Bahnen sind auch bei der Breite der Bahnen unterschiedliche Varianten möglich. So können die eine oder mehreren Bahnen jeweils eine Breite von 10 µm bis 500 µm, bevorzugt 10 µm bis 400 µm, mehr bevorzugt 10 µm bis 300 µm, noch mehr bevorzugt 10 µm bis 200 µm und besonders bevorzugt 10 µm bis 100 µm aufweisen. Eine Beschichtung mit breiteren Bahnen lässt sich gegebenenfalls leichter technisch umsetzen als eine Beschichtung mit schmaleren Bahnen. Demgegenüber kann eine Beschichtung mit schmaleren Bahnen gegebenenfalls die Zellmigration besser leiten als eine Beschichtung mit breiteren Bahnen.

Der Abstand der Bahnen voneinander kann 200 µm bis 5000 µm, bevorzugt 200 µm bis 1000 µm, mehr bevorzugt 200 µm bis 500 µm, noch mehr bevorzugt 200 µm bis 400 µm und besonders bevorzugt 200 µm bis 300 µm betragen. Umso dichter die Bahnen angeordnet sind, umso mehr Zellen können geleitet werden. Der Abstand sollte jedoch mindestens so groß gewählt werden, dass die Zellen die Bahnen nicht überspannen können. Dies sollte bei der zuvor angegebenen Untergrenze von 200 µm insbesondere bei Fibroblasten und Keratinozyten gegeben sein.

Weiterhin ist die Beschichtung bevorzugt auf maximal 10 %, 20 %, 30 %, 40 % oder 50 % der Fläche der wundzugewandten Seite der Wundkontaktschicht vorhanden. Eine vollflächige Beschichtung der wundzugewandten Seite der Wundkontaktschicht ist erfindungsgemäß nicht vorgesehen, da dabei normalerweise keine Bahnen ausgebildet werden können.

Um die Beschichtung auf die Wundkontaktschicht aufzubringen, können verschiedene Technologien zum Einsatz kommen. Zum Beispiel kann die Beschichtung mittels Mikrokontaktdruck erzeugt sein. Gemäß einer besonders bevorzugten Ausführungsform der Erfindung wird jedoch vorgeschlagen, dass die Beschichtung mittels Tintenstrahldruck (ink jet printing) erzeugt ist. Mit dem Tintenstrahldruck lassen sich bahnförmige Beschichtungen für Wundauflagen in den unterschiedlichsten Formen und Größen vergleichsweise einfach, schnell und kostengünstig realisieren. Der Tintenstrahldruck kann auch unter sterilen Bedingungen durchgeführt werden. Aufgrund der zuvor genannten Vorteile ist der Tintenstrahldruck besser für industrielle Herstellungsprozesse von Wundauflagen geeignet als der Mikrokontaktdruck. Der Tintenstrahldruck sowie die hierbei eingesetzten Geräte, die Tintenstrahldrucker, sind für sich betrachtet im Stand der Technik bekannt. Als "Tinte" dient im Zusammenhang mit der vorliegenden Erfindung die Beschichtung im flüssigen Zustand.

Bei dem Tintenstrahldruck kann es sich um einen CIJ-Druck handeln. CIJ-Druck steht dabei für "continuous ink jet"-Druck. Bei dem CIJ-Druck erzeugt der Tintenstrahldrucker kontinuierlich Tröpfchen und appliziert diese auf eine Oberfläche (vorliegend die Wundkontaktschicht). Bezogen auf die vorliegende Erfindung hat der CIJ-Druck den Vorteil, dass die Wundkontaktschicht sehr schnell beschichtet werden kann. Allerdings kann bei dem CIJ-Druck verfahrenstechnisch bedingt ein Teil der erzeugten Tröpfchen ungenutzt bleiben, was die Menge an benötigter Beschichtungslösung und damit die Kosten erhöhen kann.

Als Alternative zum CIJ-Druck ist der DOD-Druck vorgesehen. DOD-Druck steht dabei für "drop on demand"-Druck. Bei dem DOD-Druck werden die Tröpfchen nur dann erzeugt, wenn sie benötigt werden. Dadurch kann die Wundkontaktschicht sehr effizient und sparsam beschichtet werden, was für die vorliegende Erfindung als besonders vorteilhaft angesehen wird. Vorzugsweise handelt es sich um einen DOD-Druck mit mehreren Sprühdüsen. Das heißt, der eingesetzte Tintenstrahldrucker verfügt über mehrere Sprühdüsen und kann die Wundkontaktschicht dann gegebenenfalls ähnlich schnell wie ein CIJ-Drucker beschichten. Zum Beispiel kann der DOD-Drucker in dieser bevorzugten Ausführungsform der Erfindung zwei oder drei anstatt nur eine einzige Sprühdüse aufweisen.

Weitere vorteilhafte Ausführungsformen der Erfindung können im Hinblick auf die Befestigung der Beschichtung an der Wundkontaktschicht angegeben werden, wobei insbesondere davon ausgegangen wird, dass es sich bei der Wundkontaktschicht um ein Hydrogel handelt. Das zur Beschichtung verwendete Material (vorzugsweise eine Lösung, welche mittels Tintenstrahldruck auf die Wundkontaktschicht aufgebracht wird) soll eine Schicht oder Lage auf der Wundkontaktschicht ausbilden. Normalerweise ist dies eine Voraussetzung dafür, dass die Zellen mit den in der Beschichtung enthaltenen, die Zelladhäsion vermittelnden Stoffen in Kontakt treten können. Entsprechend ist es vorliegend typischerweise nicht vorgesehen, dass das zur Beschichtung verwendete Material vollständig in die Wundkontaktschicht eindringt. Jedoch kann es vorteilhaft sein, wenn die Beschichtung zumindest teilweise in die Wundkontaktschicht eindringt, um eine dauerhafte Befestigung der Beschichtung an der Wundkontaktschicht zu erreichen. Ebenso kann es vorteilhaft sein, wenn die Beschichtung im Wesentlichen nicht in die Wundkontaktschicht eindringt, sondern der Wundkontaktschicht im Wesentlichen lediglich aufgelagert ist, um den Zelladhäsion-vermittelnden Effekt der Beschichtung voll auszuschöpfen. Bei den beiden zuvor genannten Ausführungsformen wird insbesondere auf die in der Beschichtung enthaltenen, die Zelladhäsion vermittelnden Stoffe Bezug genommen. Für die Beschichtung der Wundkontaktschicht eingesetzte Hilfsstoffe ohne Zelladhäsions-vermittelnden Effekt (zum Beispiel Lösungsmittel) können bei den beiden zuvor genannten Ausführungsformen unberücksichtigt bleiben. Zum Beispiel wird im Falle einer mit einer wässrigen Fibrinogen-Lösung mittels Tintenstrahldruck beschichteten Hydrogel-Wundkontaktschicht insbesondere auf die Eindringtiefe der Fibrinogenmoleküle und nicht etwa der Wassermoleküle Bezug genommen. Ob die Beschichtung in die Wundkontaktschicht eindringt oder nicht kann im Zweifel mittels geeigneter mikroskopischer oder spektroskopischer Verfahren festgestellt werden. Geeignete mikroskopische Verfahren könnten zum Beispiel die konfokale Mikroskopie oder die Fluoreszenzmikroskopie mit entsprechenden Fluoreszenzfarbstoffen sein. Als geeignetes spektroskopisches Verfahren könnte die konfokale RAMAN Spektroskopie zum Einsatz kommen.

Eine besonders dauerhafte Befestigung der Beschichtung an der Wundkontaktschicht kann dadurch erreicht werden, indem die Beschichtung mit der Wundkontaktschicht chemisch kovalent verbunden ist. Dabei wird gleichfalls wieder insbesondere auf die in der Beschichtung enthaltenen, die Zelladhäsion vermittelnden Stoffe Bezug genommen. Es kann aber auch vorteilhaft sein, wenn die Beschichtung beziehungsweise die in der Beschichtung enthaltenen, die Zelladhäsion vermittelnden Stoffe nicht chemisch kovalent mit der Wundkontaktschicht verbunden sind, sondern lediglich an der Wundkontaktschicht zum Beispiel auf der Basis von hydrophilen, hydrophoben oder ionischen Wechselwirkungen haften. Dann kommt es zu keiner Veränderung der Beschichtung auf chemischer Ebene und die Zelladhäsions-vermittelnde Wirkung der Beschichtung kann vollständig erhalten bleiben. Zudem kann eine solche Haftung leichter technisch realisiert werden und auch überraschend stabil sein. Wenn die Beschichtung wie zuvor beschrieben lediglich an der Wundkontaktschicht haftet, findet dabei üblicherweise keine spezifische Wechselwirkung wie zum Beispiel zwischen Biotin und Avidin, Biotin und Streptavidin und/oder einem Antigen und einem Antikörper statt, um die Beschichtung an der Wundkontaktschicht zu befestigen. Demnach umfasst die Haftung üblicherweise keine Wechselwirkung zwischen Biotin und Avidin, Biotin und Streptavidin und/oder einem Antigen und einem Antikörper. Vielmehr ist eine leicht realisierbare unspezifische Haftung beabsichtigt.

Wie weit ein zur Beschichtung verwendetes Material in eine Hydrogel-Wundkontaktschicht eindringen und/oder mit dieser reagieren kann, hängt beispielsweise von den folgenden Faktoren ab:
Erstens dem Polymerisationszustand des Hydrogels, das heißt ob das Hydrogel bereits vollständig quervernetzt oder noch reaktiv ist. Letzteres begünstigt in der Regel das Eindringen des Beschichtungsmaterials in das Hydrogel sowie das Reagieren des Beschichtungsmaterials mit dem Hydrogel.
Zweitens die molekulare Struktur des (auspolymerisierten) Hydrogels. Umso weitmaschiger das Netzwerk des Hydrogels ist, umso besser sollte das Beschichtungsmaterial in das Hydrogel diffundieren und eindringen können.
Drittens das Molekulargewicht des Beschichtungsmaterials. Umso kleiner dieses ist, umso besser sollte es in das Hydrogel diffundieren und eindringen können.
Viertens die Übereinstimmung der chemischen Eigenschaften von Hydrogel und Beschichtungsmaterial. Zum Beispiel wird ein hydrophiles Beschichtungsmaterial üblicherweise besser in ein Hydrogel eindringen können als ein hydrophobes Beschichtungsmaterial. Unterschiedliche Ladungen von Hydrogel und Beschichtungsmaterial können gleichfalls dazu beitragen, dass das Beschichtungsmaterial besser in das Hydrogel eindringen kann.

Von den zuvor genannten vier Faktoren ist der erste Faktor besonders relevant. Deshalb bezieht sich eine weitere Ausgestaltung der Erfindung darauf, dass die Wundkontaktschicht ein Hydrogel umfasst oder aus einem Hydrogel besteht und die Beschichtung auf die Wundkontaktschicht, insbesondere auf das Hydrogel, zu einem Zeitpunkt aufgebracht wird, wenn das Hydrogel noch nicht vollständig quervernetzt, sondern noch reaktiv ist, so dass die Beschichtung in die Wundkontaktschicht, insbesondere in das Hydrogel, eindringen und/oder mit der Wundkontaktschicht, insbesondere mit dem Hydrogel, reagieren und sich chemisch kovalent verbinden kann. Die kovalente Verbindung der Beschichtung mit dem Hydrogel kann beispielsweise dadurch ausgebildet werden, indem Bestandteile der Beschichtung mit einer Amin- oder Hydroxyl-Gruppe mit dem zuvor erwähnten Isocyanat-terminierten Präpolymer reagieren. Viele der zuvor genannten Zelladhäsions-vermittelnden Stoffe enthalten eine Amin- oder Hydroxyl-Gruppe und sind daher in der Lage mit dem Isocyanat-terminierten Präpolymer zu reagieren, wobei eine Harnstoff-Bindung (bei Reaktion der Amin-Gruppe mit der Isocyanat-Gruppe) oder eine Urethan-Bindung (bei Reaktion der Hydroxyl-Gruppe mit der Isocyanat-Gruppe) entsteht.

Alternativ kann es vorgesehen sein, dass die Wundkontaktschicht ein Hydrogel umfasst oder aus einem Hydrogel besteht und die Beschichtung auf die Wundkontaktschicht, insbesondere auf das Hydrogel, zu einem Zeitpunkt aufgebracht wird, wenn das Hydrogel vollständig quervernetzt und nicht mehr reaktiv ist, so dass die Beschichtung nicht in die Wundkontaktschicht, insbesondere in das Hydrogel, eindringen und/oder nicht mit der Wundkontaktschicht, insbesondere mit dem Hydrogel, reagieren und sich nicht chemisch kovalent verbinden kann.

Die zuvor im Hinblick auf die Befestigung der Beschichtung an die Wundkontaktschicht beschriebenen Ausführungsformen können auch in vorteilhafter Weise miteinander kombiniert werden. Zum Beispiel kann die Beschichtung in die Wundkontaktschicht eindringen und zugleich mit dieser chemisch kovalent verbunden sein. Die Beschichtung ist dann mit der Wundkontaktschicht ganz besonders stark verbunden. Die Beschichtung kann der Wundkontaktschicht aber auch lediglich haftend aufgelagert sein, um deren Zelladhäsions-vermittelnden Effekt optimal auszunutzen. Weitere Varianten wie zum Beispiel eine in die Wundkontaktschicht eindringende, jedoch nicht mit der Wundkontaktschicht chemisch kovalent verknüpfte Beschichtung sind gleichfalls denkbar.

Die erfindungsgemäße Wundauflage umfasst mindestens eine Schicht, nämlich die Wundkontaktschicht mit der Beschichtung. Vorteilhafterweise wird die Wundauflage jedoch noch mit zusätzlichen Schichten ausgestattet. Zum Beispiel kann die Wundauflage weiterhin eine Rückschicht, insbesondere eine Rückschicht aus einer Polyurethanfolie, umfassen. Die Rückschicht kann mit einer wundabgewandten Seite der Wundkontaktschicht verbunden sein. Die Rückschicht hat im Wesentlichen die Funktion, die Wundkontaktschicht beziehungsweise alle unter ihr angeordneten Schichten der Wundauflage vor Kontamination und mechanischer Beanspruchung zu schützen.

Die Rückschicht kann auch die Aufgabe haben, die Wundauflage am Körper des Patienten zu fixieren. Hierfür kann die Rückschicht die Wundkontaktschicht überfangen und einen Kleberand ausbilden. Die Wundauflage ist dann in der Art eines Inselverbandes ausgeführt.

Darüber hinaus kann die Wundauflage weiterhin eine absorbierende Schicht, insbesondere eine absorbierende Schicht aus einem Polyurethanschaumstoff, umfassen. Damit verfügt die Wundauflage über eine größere Absorptionsfähigkeit. Die absorbierende Schicht kann mit der wundabgewandten Seite der Wundkontaktschicht verbunden sein. Die zuvor genannte Rückschicht kann dann mit einer wundabgewandten Seite der absorbierenden Schicht verbunden sein, so dass sich eine Anordnung ergibt, bei der sich die absorbierende Schicht zwischen der Wundkontaktschicht und der Rückschicht befindet.

Im Falle einer zusätzlichen Rückschicht kann das erfindungsgemäße Verfahren die weiteren, nachfolgend genannten Schritte iii. und iv. umfassen:
Verfahren zur Herstellung einer Wundauflage, umfassend die folgenden Schritte:
i. Bereitstellen einer Wundkontaktschicht.
ii. Beschichten einer wundzugewandten Seite der Wundkontaktschicht, so dass die wundzugewandte Seite der Wundkontaktschicht eine Beschichtung aufweist, wobei die Beschichtung eine Adhäsion von Zellen vermitteln kann und die Beschichtung eine oder mehrere Bahnen auf der wundzugewandten Seite der Wundkontaktschicht ausbildet.
iii. Bereitstellen einer Rückschicht, insbesondere einer Rückschicht aus einer Polyurethanfolie.
iv. Verbinden einer wundabgewandten Seite der Wundkontaktschicht mit der Rückschicht, wobei die Rückschicht die Wundkontaktschicht vorzugsweise überfängt und einen Kleberand ausbildet.

Im Falle einer zusätzlichen Rückschicht in Kombination mit einer zusätzlichen absorbierenden Schicht kann das erfindungsgemäße Verfahren die weiteren, nachfolgend genannten Schritte iii. bis vi. umfassen:
Verfahren zur Herstellung einer Wundauflage, umfassend die folgenden Schritte:
i. Bereitstellen einer Wundkontaktschicht.
ii. Beschichten einer wundzugewandten Seite der Wundkontaktschicht, so dass die wundzugewandte Seite der Wundkontaktschicht eine Beschichtung aufweist, wobei die Beschichtung eine Adhäsion von Zellen vermitteln kann und die Beschichtung eine oder mehrere Bahnen auf der wundzugewandten Seite der Wundkontaktschicht ausbildet.
iii. Bereitstellen einer Rückschicht, insbesondere einer Rückschicht aus einer Polyurethanfolie.
iv. Bereitstellen einer absorbierenden Schicht, insbesondere einer absorbierenden Schicht aus einem Polyurethanschaumstoff.
v. Verbinden einer wundabgewandten Seite der Wundkontaktschicht mit der absorbierenden Schicht.
vi. Verbinden einer wundabgewandten Seite der absorbierenden Schicht mit der Rückschicht, wobei die Rückschicht sowohl die absorbierende Schicht als auch die Wundkontaktschicht vorzugsweise überfängt und einen Kleberand ausbildet.

Ein weiterer Gegenstand der vorliegenden Erfindung ist eine Wundauflage, welche durch das erfindungsgemäße Verfahren erhältlich ist. Zudem ist ein weiterer Gegenstand der vorliegenden Erfindung eine spezielle medizinische Indikation für die erfindungsgemäße Wundauflage. Dabei wird mit der erfindungsgemäßen Wundauflage eine Wunde behandelt, welche sich in einer Granulationsphase oder einer Epithelisierungsphase befindet. Für diese beiden Phasen des Wundheilungsprozesses kann die erfindungsgemäße Wundauflage besonders gut geeignet sein.

### Beispiele und Figuren

Mit den nachfolgend beschriebenen Beispielen und Figuren soll die Erfindung exemplarisch näher erläutert und veranschaulicht werden. In den Figuren können identische Strukturelemente mit denselben Bezugszeichen ausgewiesen sein.

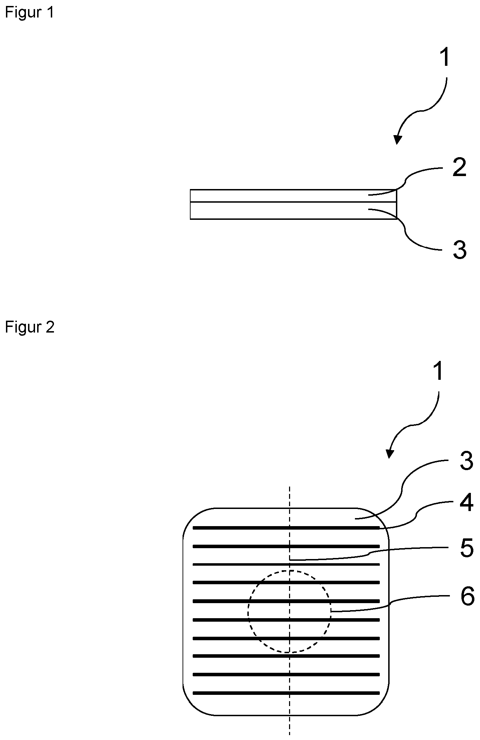

**Figur 1** zeigt eine erste Ausführungsform einer erfindungsgemäßen Wundauflage **1** von der Seite beziehungsweise in einer Schnittansicht. Die Wundauflage **1** umfasst eine Rückschicht **2.** Bei der Rückschicht **2** kann es sich um eine wasserundurchlässige, wasserdampfdurchlässige Polyurethanfolie handeln. Weiterhin umfasst die Wundauflage **1** eine Wundkontaktschicht **3,** welche vorzugsweise aus einem Hydrogel besteht. Die Wundkontaktschicht **3** weist erfindungsgemäß eine bahnförmige Beschichtung auf, an welche Zellen adhärieren können (nicht dargestellt in **Figur 1****).** Die Zelladhäsion wird durch geeignete, in der Beschichtung enthaltene Stoffe wie zum Beispiel Fibronectin, Vitronectin oder Fibrinogen vermittelt. Die Beschichtung soll dann idealerweise eine gerichtete Migration der adhärierten Zellen beispielsweise in die Wunde hinein bewirken, womit der Wundheilungsprozess beschleunigt werden soll. Geeignete Ausgestaltungen der bahnförmigen Beschichtung sind in den nachfolgenden Figuren dargestellt.
**Figur 2** zeigt eine Unterseite der Wundauflage **1.** Wie in **Figur 2** dargestellt bildet die Beschichtung **4** auf einer wundzugewandten Seite der Wundkontaktschicht **3** (Unterseite der Wundauflage **1)** ein gitterförmiges Muster mit mehreren parallelen und geradlinigen Bahnen aus. Die Bahnen verbinden zwei gegenüberliegende Randbereiche der Wundkontaktschicht **3** und verlaufen dabei über einen mittigen Bereich und zum Teil auch einen zentralen Bereich der Wundkontaktschicht **3.** Die Position des mittigen Bereichs ist in **Figur 2** mit der gestrichelten Linie **5** angedeutet. Die Position des zentralen Bereichs ist in **Figur 2** mit dem gestrichelten Kreis **6** angedeutet. Die Breite der vorzugsweise mittels DOD-Druck erzeugten Bahnen kann zum Beispiel in einem Bereich von 10 µm bis 500 µm liegen und ist in den Zeichnungen nicht maßstabsgetreu wiedergegeben. Die Abmessung der Wundauflage **1** kann beispielsweise in einem Bereich von 5 cm x 5 cm bis 20 cm x 20 cm liegen.

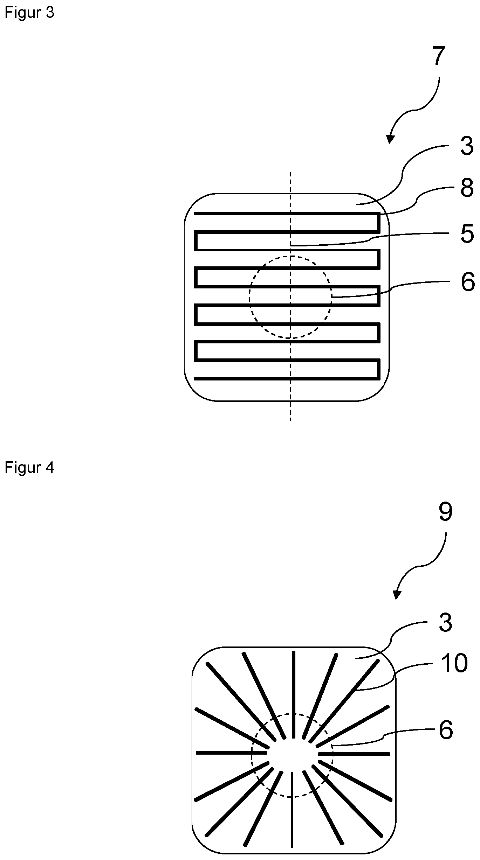

**Figur 3** zeigt eine zweite Ausführungsform einer erfindungsgemäßen Wundauflage **7** mit Blick auf eine Unterseite ähnlich wie in der vorangegangenen Figur. Im Vergleich zur Wundauflage **1** kann die Beschichtung **8** bei der Wundauflage **7** als eine einzige Bahn aufgefasst werden, welche geschlängelt über die Wundkontaktschicht **3** verläuft. Dennoch stimmt die Beschichtung **8** im Hinblick auf ihre Musterung im Wesentlichen mit der Beschichtung **4** aus **Figur 2** überein und wird vorliegend gleichfalls als gitterförmig bezeichnet.
**Figur 4** zeigt eine dritte Ausführungsform einer erfindungsgemäßen Wundauflage **9** wie zuvor mit Blick auf eine Unterseite. Die Wundauflage **9** unterscheidet sich von der Wundauflage **1** oder **7** lediglich dadurch, dass die Beschichtung **10** ein anderes Muster aufweist. Die Beschichtung **10** bildet hier ein strahlenförmiges Muster mit mehreren geradlinigen Bahnen aus, wobei die Bahnen um einen zentralen Bereich **6** der Wundkontaktschicht **3** in unterschiedliche Richtungen weisend angeordnet sind. Das strahlenförmige Muster könnte auch in leicht abgewandelter Form durch eine einzige Bahn gebildet werden, zum Beispiel wenn die Bahnen der Beschichtung **10** entsprechend miteinander verbunden werden.

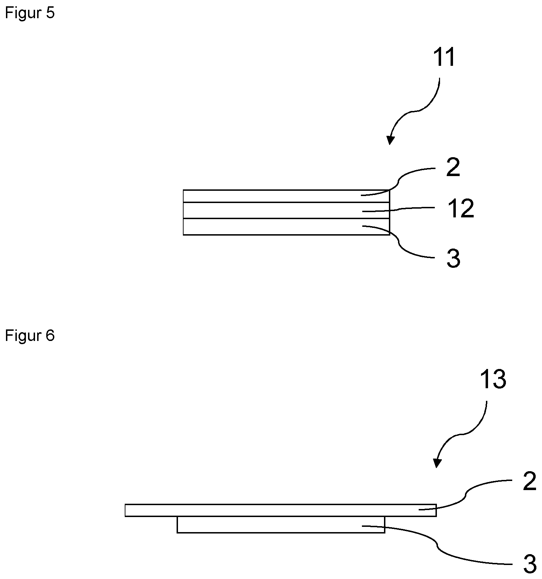

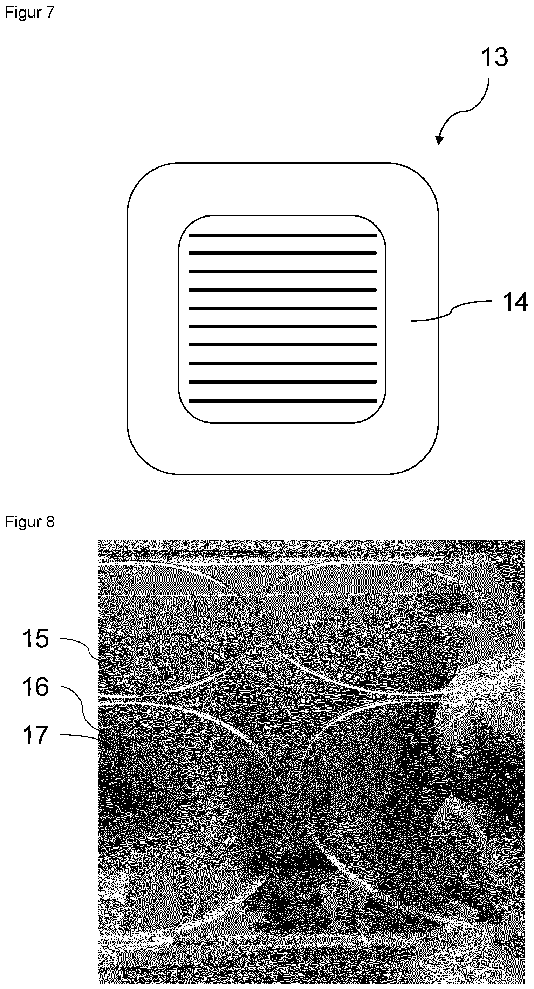

**Figur 5** zeigt eine vierte Ausführungsform einer erfindungsgemäßen Wundauflage **11** von der Seite. Anders als die Wundauflage **1, 7** oder **9** umfasst die Wundauflage **11** zusätzlich eine absorbierende Schicht **12** zwischen der Rückschicht **2** und der Wundkontaktschicht **3.** Die absorbierende Schicht **12** kann beispielsweise aus einem Polyurethanschaumstoff bestehen. Ansonsten ist die Wundauflage **11** wie die Wundauflage **1, 7** oder **9** ausgestaltet. Eine weitere Abwandlung der Wundauflage **1** als fünfte Ausführungsform einer erfindungsgemäßen Wundauflage **13** ist in den **Figuren 6** **und** **7** dargestellt. Der einzige Unterschied der Wundauflage **13** gegenüber der Wundauflage **1** besteht darin, dass die Rückschicht **2** die Wundkontaktschicht **3** überfängt und einen Kleberand **14** ausbildet. Somit entsteht ein sogenannter Inselverband. Ein zumindest im Bereich des Kleberands **14** üblicherweise vorhandener Klebstoffauftrag aus zum Beispiel einem Acrylatkleber ist in den
**Figuren 6** **und** **7** nicht dargestellt. Mit dem Kleberand **14** kann die Wundauflage sicher am Körper des Patienten befestigt werden, falls das Haftvermögen der Wundkontaktschicht **3** dafür nicht ausreicht. Dabei wird normalerweise so vorgegangen, dass der Kleberand **14** an intakter Haut in der Wundumgebung befestigt wird. Der Kleberand **14** ist nicht dafür vorgesehen, mit der Wunde in Kontakt zu treten. Hierfür ist ausschließlich die Wundkontaktschicht **3** vorgesehen. Die Wundauflage **13** kann auch wie vorangehend beschrieben mit einer zusätzlichen absorbierenden Schicht und einer strahlenförmig anstatt einer gitterförmig gemusterten Beschichtung ausgestattet werden. Die absorbierende Schicht ist dann gleichfalls bevorzugt genau so groß wie die Wundkontaktschicht **3** ausgebildet, so dass die absorbierende Schicht und die Wundkontaktschicht **3** bündig aufeinandergelegt werden können und der Kleberand **14** nicht beeinträchtigt wird.

### Stabilität einer mittels DOD-Druck erzeugten Beschichtung

Auf Glasplättchen wurde mittels Rotationsbeschichtung eine Hydrogelschicht aufgetragen. Das Hydrogel wurde durch die Umsetzung eines sechsarmigen Isocyanat-terminierten Präpolymers enthaltend Polyethylenoxideinheiten (bereitgestellt vom DWI der Rheinisch-Westfälischen Technischen Hochschule Aachen) mit Wasser erhalten. Nachdem das Hydrogel auf den Glasplättchen auspolymerisiert war, wurden Fibrinogenbahnen auf die Hydrogelschichten gedruckt. Die Druckbedingungen sind nachfolgend angegeben:
Beschichtungslösung: 10 Gew.-% Fibrinogen (F8630, Sigma-Aldrich) in PBS
DOD-Drucker: 3D Discovery, regenHU
Druck: 0,2 Bar
Vorschubgeschwindigkeit: 40 mm/min
Tröpfchenabstand: 0,2 mm
Ventilöffnungszeit: 100 µs

Ein wichtiger Parameter beim DOD-Druck ist die Tröpfchengröße. Allgemein gilt, dass sich die Tröpfchengröße vor allem durch den angelegten Druck, die Ventilöffnungszeit sowie die Viskosität der Beschichtungslösung regulieren lässt. So erhöht sich in der Regel die Tröpfchengröße, wenn der Druck oder die Ventilöffnungszeit erhöht werden. Hingegen verringert sich normalerweise die Tröpfchengröße, wenn die Viskosität der Beschichtungslösung erhöht wird. Die Viskosität kann jedoch nur in einem bestimmten Rahmen erhöht werden, da ansonsten keine Tröpfchen mehr erzeugt werden können. Für eine gegebene Tröpfchengröße ist dann der Tröpfchenabstand so zu wählen, dass Bahnen und nicht nur einzelne Punkte gedruckt werden. Die oben genannten Druckbedingungen wurden jedenfalls so gewählt, dass Fibrinogenbahnen geeigneter Breite und Qualität auf dem Hydrogel erzeugt werden konnten.

Die Hydrogel-Glasplättchen mit der gedruckten bahnförmigen Fibrinogen-Beschichtung sind in **Figur 8** zu sehen. Die Glasplättchen wurden zum Bedrucken auf den Deckel einer Six-Well-Platte gelegt und sind mit den gestrichelten Kreisen **15, 16** hervorgehoben. Die Bahnen der Beschichtung sind als helle Linien **17** erkennbar.

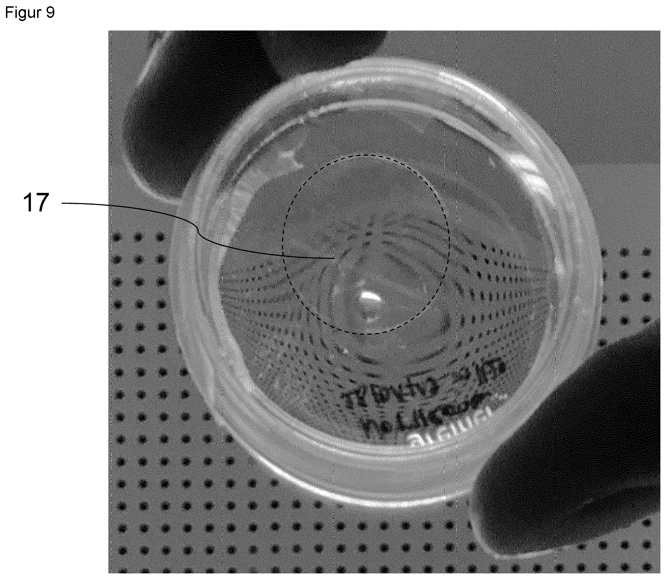

Die bedruckten Hydrogel-Glasplättchen wurden dann in Petrischalen überführt, mit demineralisiertem Wasser benetzt und für bis zu neun Tage bei 37 °C inkubiert. **Figur 9** zeigt ein Hydrogel-Glasplättchen mit der gedruckten bahnförmigen Fibrinogen-Beschichtung **17** nach neuntägiger Inkubation in Wasser. Die Fibrinogen-Beschichtung hat sich trotz der mehrtägigen Inkubation nicht von dem Hydrogel abgelöst. Dies war überraschend, da vorliegend davon ausgegangen wird, dass die Fibrinogen-Beschichtung im Wesentlichen lediglich haftend auf dem Hydrogel aufgelagert war.

### Zelladhäsion an einer mittels DOD-Druck erzeugten Beschichtung

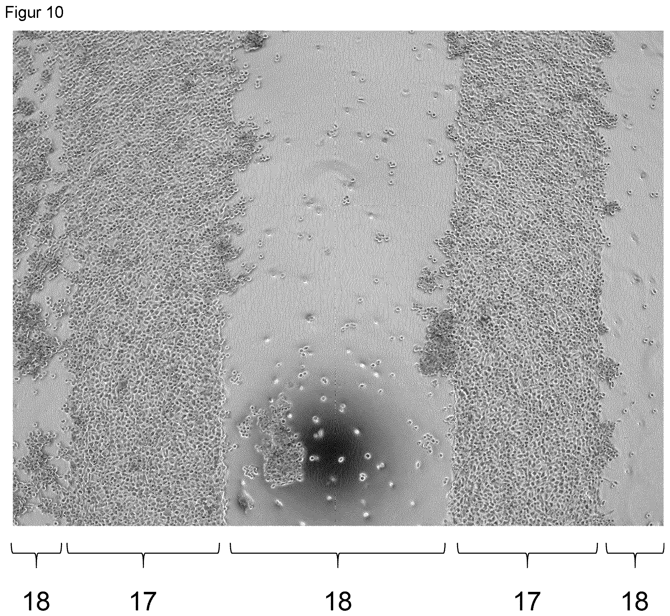

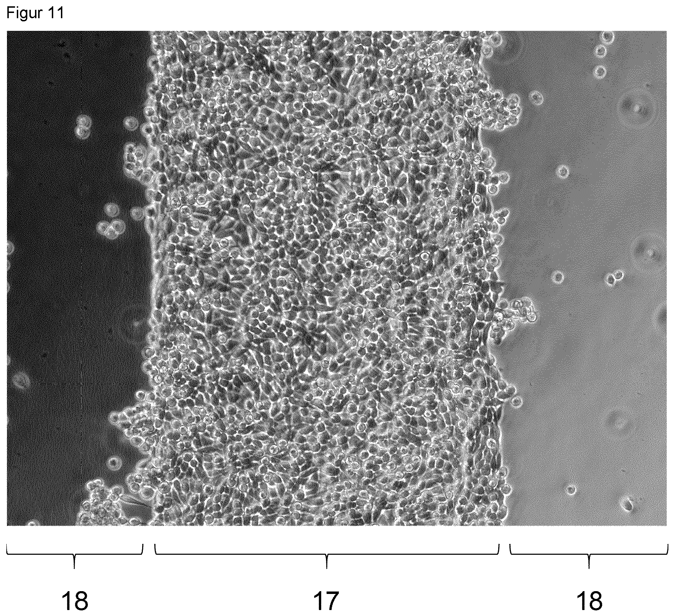

Glasplättchen wurden wie zuvor beschrieben mit einer Hydrogelschicht und einer bahnförmigen Fibrinogen-Beschichtung ausgestattet. Die bedruckten Hydrogel-Glasplättchen wurden dann in 6-Well-Platten überführt und mit adhärent wachsenden Zellen (L929 Fibroblasten) besiedelt. Nach einem Mediumwechsel mit Waschschritt wurde die Zelladhäsion mikroskopisch untersucht (siehe **Figur 10** und **Figur 11** mit einer 4-fachen beziehungsweise 10-fachen Vergrößerung). Dabei hat sich gezeigt, dass die Zellen an die Fibrinogen-Bahnen **17** gut adhärieren können, während die Zellen im Wesentlichen nicht an dem Hydrogel **18** adhärieren können.

### Erzeugen einer bahnförmigen Fibrinogen-Beschichtung mittels DOD-Druck auf einer Hydrogel-Wundauflage

Eine Wundauflage mit einer Hydrogel-Wundkontaktschicht wurde mittels DOD-Druck mit einer bahnförmigen Fibrinogen-Beschichtung versehen. Das Hydrogel wurde durch Umsetzung eines Amin-terminierten Präpolymers (Jeffamin^{®} ED-2003) mit einem dreiarmig verzweigten Isocyanat-terminierten Präpolymer (Aquapol^{®} PI-13000-31) in Gegenwart von Wasser und Glycerol erhalten. Wie bei den vorangehend beschriebenen Versuchen wurde das Hydrogel zu einem Zeitpunkt bedruckt, an dem es vollständig polymerisiert und nicht mehr reaktiv war. Die Druckbedingungen sind nachfolgend angegeben:
Beschichtungslösung: 10 Gew.-% Fibrinogen (F8630, Sigma-Aldrich) in PBS
DOD-Drucker: 3D Discovery, regenHU
Druck: 0,2 Bar
Vorschubgeschwindigkeit: 40 mm/min
Tröpfchenabstand: 0,1 mm
Ventilöffnungszeit: 100 µs

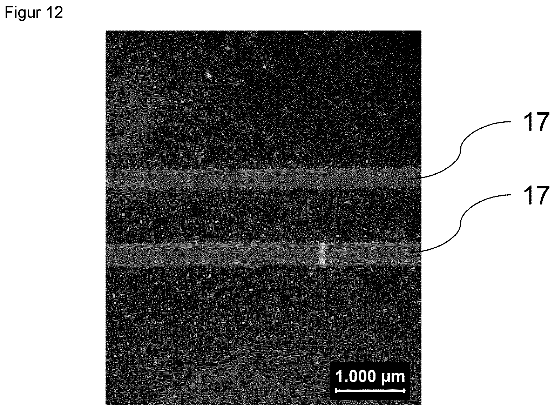

**Figur 12** zeigt dann ein vergrößertes Bild der Hydrogel-Wundkontaktschicht mit der bahnförmigen Fibrinogen-Beschichtung. Die Bahnen haben eine Breite von circa 275 µm.

Zusammenfassend haben die Versuche gezeigt, dass mittels DOD-Druck eine stabile bahnförmige Beschichtung auf Hydrogelen erzeugt werden kann. An die Beschichtung können Zellen adhärieren, wodurch sich die Möglichkeit ergibt, die Migration der Zellen in eine bestimmte Richtung zu leiten und somit eine gerichtete Migration der Zellen auszulösen.

## Patentansprüche

1. Wundauflage (1, 7, 9, 11, 13) umfassend eine Wundkontaktschicht (3), wobei eine wundzugewandte Seite der Wundkontaktschicht (3) eine Beschichtung (4, 8, 10) aufweist,
**dadurch gekennzeichnet, dass**
die Beschichtung (4, 8, 10) eine Adhäsion von Zellen vermitteln kann und die Beschichtung (4, 8, 10) eine oder mehrere Bahnen auf der wundzugewandten Seite der Wundkontaktschicht (3) ausbildet,
wobei die Wundkontaktschicht (3) ein Hydrogel umfasst oder aus einem Hydrogel besteht.

2. Verfahren zur Herstellung einer Wundauflage (1, 7, 9, 11, 13), umfassend die folgenden Schritte:
i. Bereitstellen einer Wundkontaktschicht (3), wobei die Wundkontaktschicht (3) ein Hydrogel umfasst oder aus einem Hydrogel besteht,
ii. Beschichten einer wundzugewandten Seite der Wundkontaktschicht (3), so dass die wundzugewandte Seite der Wundkontaktschicht (3) eine Beschichtung (4, 8, 10) aufweist, wobei die Beschichtung (4, 8, 10) eine Adhäsion von Zellen vermitteln kann und die Beschichtung (4, 8, 10) eine oder mehrere Bahnen auf der wundzugewandten Seite der Wundkontaktschicht (3) ausbildet.

3. Wundauflage oder Verfahren nach einem der vorangehenden Ansprüche, wobei das Hydrogel erhältlich ist durch Umsetzung eines Amin-terminierten Präpolymers enthaltend Polyalkylenoxideinheiten mit einem Isocyanat-terminierten Präpolymer enthaltend Polyalkylenoxideinheiten, wobei die Umsetzung vorzugsweise in Gegenwart eines mehrwertigen Alkohols, insbesondere Ethylenglykol, Glycerol, Sorbitol, PEG300, PEG2000 oder Saccharose, und vorzugsweise in Gegenwart von Wasser erfolgt.

4. Wundauflage oder Verfahren nach einem der vorangehenden Ansprüche, wobei die Beschichtung (4, 8, 10) eine Migration und/oder Proliferation von Zellen leiten und/oder fördern kann, wobei es sich bei den Zellen vorzugsweise um an der Wundheilung beteiligte Zellen, insbesondere um Fibroblasten oder Keratinozyten, handelt.

5. Wundauflage oder Verfahren nach einem der vorangehenden Ansprüche, wobei die Beschichtung (4, 8, 10)
- einen Alkylrest, insbesondere einen Alkylrest mit mindestens 8 Kohlenstoffatomen, und/oder
- einen Alkohol, insbesondere einen Alkohol mit mindestens 8 Kohlenstoffatomen, und/oder
- ein Amin, insbesondere ein Amin mit mindestens 8 Kohlenstoffatomen, und/oder
- eine Fettsäure, insbesondere eine mittelkettige oder langkettige Fettsäure, und/oder
- ein Bestandteil einer extrazellulären Matrix, und/oder
- ein denaturierten Bestandteil einer extrazellulären Matrix, insbesondere Gelatine, und/oder
- ein Gerinnungsfaktor, insbesondere Fibrinogen, und/oder
- ein Zelladhäsionsmolekül, und/oder
- ein synthetisches Polymer, insbesondere Polylysin, und/oder
- einen Zucker, insbesondere ein Oligosaccharid oder ein Polysaccharid,
umfasst,
wobei der Alkohol vorzugsweise Tridecan-1-ol, Tetradecan-1-ol, Pentadecan-1-ol, Hexadecan-1-ol oder Octadecan-1-ol ist,
wobei die Fettsäure vorzugsweise Tridecansäure, Tetradecansäure, Pentadecansäure, Hexadecansäure, Heptadecansäure, Octadecansäure, Ölsäure, Linolsäure oder Linolensäure ist,
wobei der Bestandteil einer extrazellulären Matrix vorzugsweise Elastin, Entactin, Fibronectin, Hyaluronsäure, Kollagen, Laminin oder Vitronectin ist, und
wobei das Zelladhäsionsmolekül vorzugsweise ein Cadherin, ein Integrin oder ein Selektin ist.

6. Wundauflage oder Verfahren nach einem der vorangehenden Ansprüche, wobei die eine oder mehreren Bahnen von einem Randbereich der wundzugewandten Seite der Wundkontaktschicht (3) mindestens zu einem mittigen Bereich (5) oder zentralen Bereich (6) der wundzugewandten Seite der Wundkontaktschicht (3) verlaufen.

7. Wundauflage oder Verfahren nach einem der vorangehenden Ansprüche, wobei die eine oder mehreren Bahnen ein gitterförmiges Muster (4, 8), sternförmiges Muster oder strahlenförmiges Muster (10) auf der wundzugewandten Seite der Wundkontaktschicht (3) ausbilden.

8. Wundauflage oder Verfahren nach einem der vorangehenden Ansprüche, wobei die eine oder mehreren Bahnen jeweils eine Breite von 10 µm bis 500 µm, bevorzugt 10 µm bis 400 µm, mehr bevorzugt 10 µm bis 300 µm, noch mehr bevorzugt 10 µm bis 200 µm und besonders bevorzugt 10 µm bis 100 µm aufweisen.

9. Wundauflage oder Verfahren nach einem der vorangehenden Ansprüche, wobei die Beschichtung (4, 8, 10) auf maximal 10 %, 20 %, 30 %, 40 % oder 50 % der Fläche der wundzugewandten Seite der Wundkontaktschicht (3) vorhanden ist.

10. Wundauflage oder Verfahren nach einem der vorangehenden Ansprüche, wobei die Beschichtung (4, 8, 10) mittels Tintenstrahldruck erzeugt ist, wobei es sich vorzugsweise um einen CIJ-Druck oder einen DOD-Druck, insbesondere einen DOD-Druck mit mehreren Sprühdüsen, handelt.

11. Wundauflage oder Verfahren nach einem der vorangehenden Ansprüche, wobei
- die Beschichtung (4, 8, 10) teilweise in die Wundkontaktschicht (3) eindringt oder
- die Beschichtung (4, 8, 10) im Wesentlichen nicht in die Wundkontaktschicht (3) eindringt, sondern der Wundkontaktschicht (3) im Wesentlichen lediglich aufgelagert ist.

12. Wundauflage oder Verfahren nach einem der vorangehenden Ansprüche, wobei
- die Beschichtung (4, 8, 10) mit der Wundkontaktschicht (3) chemisch kovalent verbunden ist oder
- die Beschichtung (4, 8, 10) mit der Wundkontaktschicht (3) nicht chemisch kovalent verbunden ist, sondern lediglich an der Wundkontaktschicht (3) haftet.

13. Wundauflage oder Verfahren nach einem der vorangehenden Ansprüche, wobei
- die Wundkontaktschicht (3) ein Hydrogel umfasst oder aus einem Hydrogel besteht und die Beschichtung (4, 8, 10) auf die Wundkontaktschicht (3) aufgebracht wird, wenn das Hydrogel noch nicht vollständig quervernetzt, sondern noch reaktiv ist, so dass die Beschichtung (4, 8, 10) in die Wundkontaktschicht (3) eindringen und/oder mit der Wundkontaktschicht (3) reagieren und sich chemisch kovalent verbinden kann, oder
- die Wundkontaktschicht (3) ein Hydrogel umfasst oder aus einem Hydrogel besteht und die Beschichtung (4, 8, 10) auf die Wundkontaktschicht (3) aufgebracht wird, wenn das Hydrogel vollständig quervernetzt und nicht mehr reaktiv ist, so dass die Beschichtung (4, 8, 10) nicht in die Wundkontaktschicht (3) eindringen und/oder nicht mit der Wundkontaktschicht (3) reagieren und sich nicht chemisch kovalent verbinden kann.

14. Wundauflage oder Verfahren nach einem der vorangehenden Ansprüche, wobei die Wundauflage (1, 7, 9, 11, 13) weiterhin eine Rückschicht (2), insbesondere eine Rückschicht (2) aus einer Polyurethanfolie, umfasst, wobei die Rückschicht (2) vorzugsweise die Wundkontaktschicht (3) überfängt und einen Kleberand (14) ausbildet.

15. Wundauflage oder Verfahren nach einem der vorangehenden Ansprüche, wobei die Wundauflage (11) weiterhin eine absorbierende Schicht (12), insbesondere eine absorbierende Schicht (12) aus einem Polyurethanschaumstoff, umfasst.

## Claims

1. Wound dressing (1, 7, 9, 11, 13) comprising a wound contact layer (3), wherein a wound-facing side of the wound contact layer (3) has a coating (4, 8, 10),
**characterized in that**
the coating (4, 8, 10) is able to mediate adhesion of cells and the coating (4, 8, 10) forms one or more lanes on the wound-facing side of the wound contact layer (3), wherein the wound contact layer (3) comprises a hydrogel or consists of a hydrogel.

2. Method for producing a wound dressing (1, 7, 9, 11, 13), comprising the following steps:
i. providing a wound contact layer (3), wherein the wound contact layer (3) comprises a hydrogel or consists of a hydrogel,
ii. coating a wound-facing side of the wound contact layer (3), so that the wound-facing side of the wound contact layer (3) has a coating (4, 8, 10), wherein the coating (4, 8, 10) is able to mediate adhesion of cells and the coating (4, 8, 10) forms one or more lanes on the wound-facing side of the wound contact layer (3).

3. Wound dressing or method according to any of the preceding claims, wherein the hydrogel is obtainable by reaction of an amine-terminated prepolymer containing polyalkylene oxide units with an isocyanate-terminated prepolymer containing polyalkylene oxide units, wherein the reaction takes place preferably in the presence of a polyhydric alcohol, more particularly ethylene glycol, glycerol, sorbitol, PEG300, PEG2000 or sucrose, and preferably in the presence of water.

4. Wound dressing or method according to any of the preceding claims, wherein the coating (4, 8, 10) is able to guide and/or promote migration and/or proliferation of cells, wherein the cells are preferably cells involved in wound healing, more particularly fibroblasts or keratinocytes.

5. Wound dressing or method according to any of the preceding claims, wherein the coating (4, 8, 10) comprises
- an alkyl radical, more particularly an alkyl radical having at least 8 carbon atoms, and/or
- an alcohol, more particularly an alcohol having at least 8 carbon atoms, and/or
- an amine, more particularly an amine having at least 8 carbon atoms, and/or
- a fatty acid, more particularly a medium-chain or long-chain fatty acid, and/or
- a constituent of an extracellular matrix, and/or
- a denatured constituent of an extracellular matrix, more particularly gelatine, and/or
- a coagulation factor, more particularly fibrinogen, and/or
- a cell adhesion molecule, and/or
- a synthetic polymer, more particularly polylysine, and/or
- a sugar, more particularly an oligosaccharide or a polysaccharide,
wherein the alcohol is preferably tridecan-1-ol, tetradecan-1-ol, pentadecan-1-ol, hexadecan-1-ol or octadecan-1-ol,
wherein the fatty acid is preferably tridecanoic acid, tetradecanoic acid, pentadecanoic acid, hexadecanoic acid, heptadecanoic acid, octadecanoic acid, oleic acid, linoleic acid or linolenic acid,
wherein the constituent of an extracellular matrix is preferably elastin, entactin, fibronectin, hyaluronic acid, collagen, laminin or vitronectin, and
wherein the cell adhesion molecule is preferably a cadherin, an integrin or a selectin.

6. Wound dressing or method according to any of the preceding claims, wherein the one or more lanes extend from an edge region of the wound-facing side of the wound contact layer (3) at least to a middle region (5) or central region (6) of the wound-facing side of the wound contact layer (3).

7. Wound dressing or method according to any of the preceding claims, wherein the one or more lanes form a lattice-like pattern (4, 8), star-shaped pattern or radiant pattern (10) on the wound-facing side of the wound contact layer (3).

8. Wound dressing or method according to any of the preceding claims, wherein the one or more lanes each have a width of 10 µm to 500 µm, preferably 10 µm to 400 µm, more preferably 10 µm to 300 µm, even more preferably 10 µm to 200 µm and very preferably 10 µm to 100 µm.

9. Wound dressing or method according to any of the preceding claims, wherein the coating (4, 8, 10) is present on a maximum of 10%, 20%, 30%, 40% or 50% of the area of the wound-facing side of the wound contact layer (3).

10. Wound dressing or method according to any of the preceding claims, wherein the coating (4, 8, 10) is produced by means of inkjet printing, which is preferably CIJ printing or DOD printing, more particularly DOD printing with multiple spray nozzles.

11. Wound dressing or method according to any of the preceding claims, wherein
- the coating (4, 8, 10) penetrates partially into the wound contact layer (3) or
- the coating (4, 8, 10) substantially does not penetrate into the wound contact layer (3), but is substantially merely supported on the wound contact layer (3).

12. Wound dressing or method according to any of the preceding claims, wherein
- the coating (4, 8, 10) is chemically covalently bonded to the wound contact layer (3) or
- the coating (4, 8, 10) is not chemically covalently bonded to the wound contact layer (3), but merely adheres to the wound contact layer (3).

13. Wound dressing or method according to any of the preceding claims, wherein
- the wound contact layer (3) comprises a hydrogel or consists of a hydrogel and the coating (4, 8, 10) is applied to the wound contact layer (3) when the hydrogel is not yet completely crosslinked but is still reactive, so that the coating (4, 8, 10) can penetrate into the wound contact layer (3) and/or react with and chemically covalently bond to the wound contact layer (3), or
- the wound contact layer (3) comprises a hydrogel or consists of a hydrogel and the coating (4, 8, 10) is applied to the wound contact layer (3) when the hydrogel is completely crosslinked and no longer reactive, so that the coating (4, 8, 10) cannot penetrate into the wound contact layer (3) and/or cannot react with and chemically covalently bond to the wound contact layer (3).

14. Wound dressing or method according to any of the preceding claims, wherein the wound dressing (1, 7, 9, 11, 13) further comprises a backing layer (2), more particularly a backing layer (2) composed of a polyurethane film, wherein the backing layer (2) preferably overhangs the wound contact layer (3) and forms an adhesive edge (14).

15. Wound dressing or method according to any of the preceding claims, wherein the wound dressing (11) further comprises an absorbent layer (12), more particularly an absorbent layer (12) composed of a polyurethane foam.

## Revendications

1. Pansement (1, 7, 9, 11, 13) comprenant une couche de contact (3) avec la plaie, une face orientée vers la plaie de la couche de contact (3) avec la plaie présentant un revêtement (4, 8, 10),
**caractérisé en ce que**
le revêtement (4, 8, 10) peut promouvoir une adhérence de cellules et le revêtement (4, 8, 10) forme une ou plusieurs bandes sur la face orientée vers la plaie de la couche de contact (3) avec la plaie,
la couche de contact (3) avec la plaie comprenant un hydrogel ou étant constituée par un hydrogel.

2. Procédé de fabrication d'un pansement (1, 7, 9, 11, 13) comprenant les étapes suivantes :
i. mise à disposition d'une couche de contact (3) avec la plaie, la couche de contact (3) avec la plaie comprenant un hydrogel ou étant constituée par un hydrogel,
ii. revêtement d'une face orientée vers la plaie de la couche de contact (3) avec la plaie, de telle sorte que la face orientée vers la plaie de la couche de contact (3) avec la plaie présente un revêtement (4, 8, 10), le revêtement (4, 8, 10) pouvant promouvoir une adhérence de cellules et le revêtement (4, 8, 10) formant une ou plusieurs bandes sur la face orientée vers la plaie de la couche de contact (3) avec la plaie.

3. Pansement ou procédé selon l'une des revendications précédentes, l'hydrogel pouvant être obtenu par transformation d'un prépolymère terminé par amine contenant des motifs poly(oxyde d'alkylène) avec un prépolymère terminé par isocyanate contenant des motifs poly(oxyde d'alkylène), la transformation ayant de préférence lieu en présence d'un alcool polyvalent, en particulier l'éthylèneglycol, le glycérol, le sorbitol, le PEG300, le PEG2000 ou le saccharose et de préférence en présence d'eau.

4. Pansement ou procédé selon l'une des revendications précédentes, le revêtement (4, 8, 10) pouvant guider et/ou favoriser une migration et/ou une prolifération de cellules, les cellules étant de préférence des cellules participant à la guérison de la plaie, en particulier des fibroblastes ou des kératinocytes.

5. Pansement ou procédé selon l'une des revendications précédentes, le revêtement (4, 8, 10) comprenant
- un radical alkyle, en particulier un radical alkyle comprenant au moins 8 atomes de carbone et/ou
- un alcool, en particulier un alcool comprenant au moins 8 atomes de carbone et/ou
- une amine, en particulier une amine comprenant au moins 8 atomes de carbone et/ou
- un acide gras, en particulier un acide gras à chaîne moyenne ou à longue chaîne et/ou
- un constituant d'une matrice extracellulaire et/ou
- un constituant dénaturé d'une matrice extracellulaire, en particulier la gélatine et/ou
- un facteur de coagulation, en particulier le fibrinogène et/ou
- une molécule d'adhérence cellulaire et/ou
- un polymère synthétique, en particulier la polylysine et/ou
- un sucre, en particulier un oligosaccharide ou une polysaccharide,
l'alcool étant de préférence le tridécan-1-ol, le tétradécan-1-ol, le pentadécan-1-ol, l'hexadécan-1-ol ou l'octadécan-1-ol,
l'acide gras étant de préférence l'acide tridécanoïque, l'acide tétradécanoïque, l'acide pentadécanoïque, l'acide hexadécanoïque, l'acide heptadécanoïque, l'acide octadécanoïque, l'acide oléique, l'acide linoléique ou l'acide linolénique,
le constituant d'une matrice extracellulaire étant de préférence l'élastine, l'entactine, la fibronectine, l'acide hyaluronique, le collagène, la laminine ou la vitronectine et
la molécule d'adhérence cellulaire étant de préférence une cadhérine, une intégrine ou une sélectine.

6. Pansement ou procédé selon l'une des revendications précédentes, ladite une ou lesdites plusieurs bandes s'étendant à partir d'une zone de bord de la face orientée vers la plaie de la couche de contact (3) avec la plaie, au moins vers une zone médiane (5) ou une zone centrale (6) de la face orientée vers la plaie de la couche de contact (3) avec la plaie.

7. Pansement ou procédé selon l'une des revendications précédentes, ladite une ou lesdites plusieurs bandes formant un modèle en forme de grille (4, 8), un modèle en forme d'étoile ou une modèle en forme de rayons (10) sur la face orientée vers la plaie de la couche de contact (3) avec la plaie.

8. Pansement ou procédé selon l'une des revendications précédentes, ladite une ou lesdites plusieurs bandes présentant à chaque fois une largeur de 10 µm à 500 µm, de préférence de 10 µm à 400 µm, plus préférablement de 10 µm à 300 µm, encore plus préférablement de 10 µm à 200 µm et de manière particulièrement préférée de 10 µm à 100 µm.

9. Pansement ou procédé selon l'une des revendications précédentes, le revêtement (4, 8, 10) étant présent sur au maximum 10%, 20%, 30%, 40% ou 50% de la surface de la face orientée vers la plaie de la couche de contact (3) avec la plaie.

10. Pansement ou procédé selon l'une des revendications précédentes, le revêtement (4, 8, 10) étant généré par impression par jet d'encre, l'impression étant de préférence une impression CIJ (à jet d'encre continu) ou une impression DOD (jet à la demande), en particulier une impression DOD à l'aide de plusieurs buses de pulvérisation.

11. Pansement ou procédé selon l'une des revendications précédentes,
- le revêtement (4, 8, 10) pénétrant partiellement dans la couche de contact (3) avec la plaie ou
- le revêtement (4, 8, 10) ne pénétrant sensiblement pas dans la couche de contact (3) avec la plaie mais étant simplement placé sur la couche de contact (3) avec la plaie.

12. Pansement ou procédé selon l'une des revendications précédentes,
- le revêtement (4, 8, 10) étant relié chimiquement par covalence avec la couche de contact (3) avec la plaie ou
- le revêtement (4, 8, 10) n'étant pas relié chimiquement par covalence avec la couche de contact (3) avec la plaie, mais adhérant simplement à la couche de contact (3) avec la plaie.

13. Pansement ou procédé selon l'une des revendications précédentes,
- la couche de contact (3) avec la plaie comprenant un hydrogel ou étant constituée par un hydrogel et le revêtement (4, 8, 10) étant appliqué sur la couche de contact (3) avec la plaie lorsque l'hydrogel n'est pas encore complètement réticulé, mais est encore réactif, de telle sorte que le revêtement (4, 8, 10) peut pénétrer dans la couche de contact (3) avec la plaie et/ou peut réagir et se lier chimiquement par covalence avec la couche de contact (3) avec la plaie ou
- la couche de contact (3) avec la plaie comprenant un hydrogel ou étant constituée par un hydrogel et le revêtement (4, 8, 10) étant appliqué sur la couche de contact (3) avec la plaie lorsque l'hydrogel est complètement réticulé et n'est plus réactif, de telle sorte que le revêtement (4, 8, 10) ne peut pas pénétrer dans la couche de contact (3) avec la plaie et/ou ne peut pas réagir ni se lier chimiquement par covalence avec la couche de contact (3) avec la plaie.

14. Pansement ou procédé selon l'une des revendications précédentes, le pansement (1, 7, 9, 11, 13) comprenant en outre une couche arrière (2), en particulier une couche arrière (2) en une feuille de polyuréthane, la couche arrière (2) recouvrant de préférence la couche de contact (3) avec la plaie et formant un bord collant (14).

15. Pansement ou procédé selon l'une des revendications précédentes, le pansement (11) comprenant en outre une couche absorbante (12), en particulier une couche absorbante (12) en une mousse de polyuréthane.
